# EUROPEAN PATENT APPLICATION

(11) **EP 3 779 410 A1**
(43) Date of publication of application: **17.02.2021**
(21) Application number: 19775438.5
(22) Date of filing: 20.03.2019
(51) Int. Cl.: G01N 21/64, C12M 1/34, G01N 33/48, G01N 33/483, G06T 7/90

(54) **IMAGE PROCESSING METHOD, IMAGE PROCESSING DEVICE, AND PROGRAM**

(30) Priority: 30.03.2018 JP 2018066856
(71) Applicant: KONICA MINOLTA, INC., Tokyo 100-7015 (JP)
(72) Inventor: KAZAYAMA, Yuki, Tokyo 100-7015 (JP); YAMAZAKI, Ryo, Tokyo 100-7015 (JP)
(74) Representative: Henkel & Partner mbB
(86) International application number: PCT/JP2019/011661
(87) International publication number: WO 2019/188647

(57) **Abstract**

The present invention addresses the problem of providing an image processing method, an image processing device, and a program, with which it is possible for the type of a cell in a complex stained state to be identified, in cell identification employing image recognition technology. An image processing method for identifying the type of a cell on the basis of differences between stained states of cells, from a multi-stained image obtained by capturing an image of a tissue specimen stained in multiple using a plurality of dyes having different coloring, is provided with a staining information identifying step of identifying a cell candidate region in which a cell in a specific stained state is present in the multi-stained image, and a region forming step of forming a cell region by subjecting the cell candidate region to a prescribed region forming process, wherein the staining information identifying step involves identifying the stained state of the cell in the multi-stained image on the basis of a spatial correlation between the dyes and a relative positional relationship between constituent elements of the cell, calculating a probability of presence of the cell in the specific stained state for each position in the multi-stained image, and outputting a probability map in which the probabilities of presence are connected together spatially.

## Description

### TECHNOLOGICAL FIELD

The present invention relates to an image processing method, an image processing device, and a program.

### BACKGROUND ART

In pathological diagnosis, specification of a specific biological material, such as a protein, overexpressing in a tissue section and an expression level thereof provides very important information for predicting a prognosis and for deciding a treatment plan afterward.

In recent years, a technology of combining a fluorescence image obtained through a fluorescent staining technology of staining a specific biological material using a fluorescent substance, for example, fluorescent in situ hybridization (FISH) and a morphological cell image obtained by staining cells using an existing immunostaining technology, and performing a protein expression level analysis and a molecular conductor analysis on the cell level is being developed. Since this requires cell types to be identified correctly in the morphological cell image, a determination should be made indispensably by a specialist such as a pathologist. However, it requires time to manually observe a huge number of cells in a tissue section, and to identify their types.

Therefore, in recent years, many techniques of automatically identifying cell regions in a cell image using an image recognition technology are being studied.

For example, Patent Document 1 discloses a method of automatically extracting the cell outline using a nucleus stained image and a cell membrane stained image captured by imaging cells subjected to multiple staining with a labeled specific binding partner for the nucleus and a labeled specific binding partner for the cell membrane in the same field of view. By automatically performing cell identification or the like in this manner using the image recognition technology without human intervention, burdens on the medical field will be significantly reduced.

### PRIOR ART DOCUMENT

### PATENT DOCUMENT

Patent Document 1: JP 2011-186750 A

### SUMMARY OF THE INVENTION

### PROBLEMS TO BE SOLVED BY THE INVENTION

In some cases, in a multiple-stained image obtained by subjecting cells to multiple staining using stains targeting on a plurality of types of biological materials in the cells, cell types are identified based on the difference in stained state.

For example, macrophages are cells that form a cancer microenvironment, and some macrophages are known to shift from an anti-tumor M1 type to a tumor-promoting M2 type in the cancer microenvironment. They are called tumor-associated macrophages (TAM), which are considered as being an effective target in tumor treatment. In order to identify the presence of TAM in a tissue section, multiple staining targeting on a protein specifically expressed in the M1 type macrophages and a protein specifically expressed in the M2 type macrophages, respectively, is performed to identify TAM based on the difference in stained state.

In the method described in Patent Document 1, it is possible to extract cells from a morphological cell image, whereas it is difficult to identity complicated identification targets as described above, that is, cells in a fragmentary stained state, such as single-positive cells stained only with a single stain or multi-positive cells stained with a plurality of stains, based on the difference in stained state.

The present invention was made in view of the above problem, and has an object to provide an image processing method, an image processing device, and a program that, in cell identification through use of an image recognition technology, enable types of cells in a complicated stained state to be identified.

### MEANS FOR SOLVING PROBLEMS

To solve the above problem, the image processing method according to claim 1 is an image processing method for identifying cell types in a multiple-stained image obtained by imaging a tissue specimen subjected to multiple staining with a plurality of stains that produce different colors, based on a difference in stained state between cells, including:
identifying staining information by identifying a cell candidate region in which cells in a specific stained state exist in the multiple-stained image; and
forming a region by subjecting the cell candidate region to predetermined region forming processing to form a cell region in which the cells in the specific stained state have been identified,
wherein identifying the staining information includes:
   identifying a state of the cells in the multiple-stained image based on:
      a spatial correlation between the stains representing whether staining of a pixel, positioned in proximity to a pixel detected as having been stained with one stain, with another stain has been detected; and
      a relative positional relationship between cell components; and
   calculating existence probabilities of the cells in the specific stained state for respective positions in the multiple-stained image, and outputting a probability map obtained by spatially connecting the existence probabilities.

The invention according to claim 2 is the image processing method according to claim 1, wherein the tissue specimen includes a cell in which a plurality of different biological materials have been stained with stains that produce different colors, respectively.

The invention according to claim 3 is the image processing method according to claim 1 or 2, wherein forming the region includes, as the predetermined region forming processing, subjecting the cell candidate region to removal of a small-area region and/or interpolation of a lost region.

The invention according to claim 4 is the image processing method according to any one of claims 1 to 3, including:
identifying division information by identifying a division reference for dividing the cell candidate region into predetermined regions,
wherein identifying the division information includes:
   calculating existence probabilities of the division reference for respective positions in the multiple-stained image based on the spatial correlation between the plurality of stains and the relative positional relationship between the cell components; and
   generating a probability map of the division reference obtained by spatially connecting the existence probabilities.

The invention according to claim 5 is the image processing method according to claim 4, wherein the division reference is a nucleus located inside a region stained with the stains.

The invention according to claim 6 is the image processing method according to claim 4, wherein the division reference is an unstained region located inside the cell candidate region and surrounded by a region stained with the stains.

The invention according to claim 7 is the image processing method according to any one of claims 4 to 6, wherein forming the region includes, as the predetermined region forming processing, dividing the cell candidate region output in identifying the staining information using the division reference output in identifying the division information as a reference.

The invention according to claim 8 is an image processing device that identifies cell types in a multiple-stained image obtained by imaging a tissue specimen subjected to multiple staining with a plurality of stains that produce different colors, based on a difference in stained state between cells, including:
a staining information identifier that identifies a cell candidate region in which cells in a specific stained state exist in the multiple-stained image; and
a region forming unit that subjects the cell candidate region to predetermined region forming processing to form a cell region in which the cells in the specific stained state have been identified,
wherein the staining information identifier
identifies the stained state of the cells in the multiple-stained image based on:
   a spatial correlation between the stains representing whether staining of a pixel, positioned in proximity to a pixel detected as having been stained with one stain, with another stain has been detected; and
   a relative positional relationship between cell components, and
calculates existence probabilities of the cells in the specific stained state for respective positions in the multiple-stained image, and outputs a probability map obtained by spatially connecting the existence probabilities.

The invention according to claim 9 is a program that makes a computer in an image processing device that identifies cell types in a multiple-stained image obtained by imaging a tissue specimen subjected to multiple staining with a plurality of stains that produce different colors based on a difference in stained state between cells function as:
a staining information identifier that identifies a cell candidate region in which cells in a specific stained state exist in the multiple-stained image; and
a region forming unit that subjects the cell candidate region to predetermined region forming processing to form a cell region in which the cells in the specific stained state have been identified,
wherein the program causes the staining information identifier to
identify the stained state of the cells in the multiple-stained image based on:
   a spatial correlation between the stains representing whether staining of a pixel, positioned in proximity to a pixel detected as having been stained with one stain, with another stain has been detected; and
   a relative positional relationship between cell components, and
calculate existence probabilities of the cells in the specific stained state for respective positions in the multiple-stained image, and output a probability map obtained by spatially connecting the existence probabilities.

### ADVANTAGEOUS EFFECTS OF INVENTION

The present invention provides an image processing method, an image processing device, and a program that, in cell identification through use of an image recognition technology, enable types of cells in a complicated stained state to be identified.

### BRIEF DESCRIPTION OF THE DRAWINGS

FIG. 1 shows a system configuration of an image processing system to which an image processing method of the present invention is applied.
FIG. 2 is a block diagram showing a functional configuration of an image processing device in FIG. 1.
FIG. 3 is a flow chart showing cell identification processing 1 according to the first embodiment.
FIG. 4 is a flow chart showing staining information identification processing according to the first embodiment.
FIG. 5A shows a concept of a probability map.
FIG. 5B shows a concept of a probability map.
FIG. 6 is a flow chart showing region forming processing according to the first embodiment.
FIG. 7 is a flow chart showing cell identification processing 2 according to the second embodiment.
FIG. 8 is a flow chart showing division information identification processing according to the second embodiment.
FIG. 9 is a flow chart showing region forming processing according to the second embodiment.
FIG. 10 shows an example of output obtained by cell identification processing 2 in the second embodiment.

### EMBODIMENTS FOR CARRYING OUT THE INVENTION

### FIRST EMBODIMENT

Hereinafter, the first embodiment for carrying out the present invention will be described with reference to the drawings.

### CONFIGURATION OF IMAGE PROCESSING SYSTEM 100

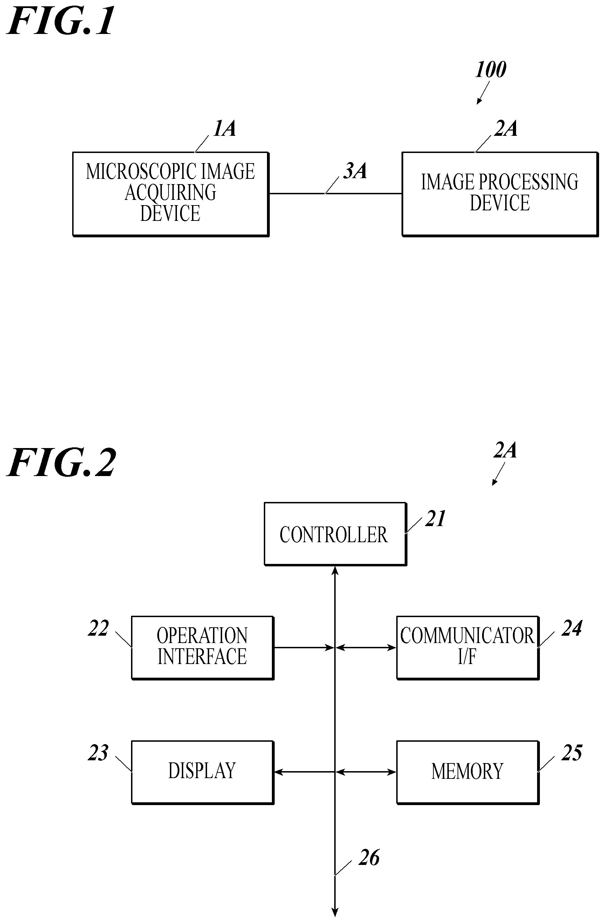

FIG. 1 shows an overall configuration example of an image processing system 100 according to the present invention. The image processing system 100 acquires a microscopic image of a tissue specimen stained with predetermined stains and analyzes the acquired microscopic image. The system identifies and outputs cell types in the tissue specimen.

As shown in FIG. 1, an image processing system 100 is configured such that the microscopic image acquiring device 1A and the image processing device 2A are connected so as to be able to transmit and receive data via an interface, such as a cable 3A. The connection between the microscope image acquiring device 1A and the image processing device 2A is not particularly limited. For example, the microscope image acquiring device 1A and the image processing device 2A may be connected via a LAN (Local Area Network) or may be connected wirelessly.

The microscopic image acquiring device 1A is a well-known optical microscope with a camera which obtains the microscopic image of the tissue specimen on a slide placed on a slide fixing stage and sends it to the image processing device 2A.

The microscopic image acquiring device 1A includes an irradiating unit, an image forming unit, an imaging unit, a communicator I/F, and the like. The irradiating unit includes a light source, a filter, and the like, and irradiates the tissue specimen on the slide placed on the slide fixing stage with light. The image forming unit includes an ocular lens, an object lens, and the like, and forms an image of transmitted light and reflected light from the tissue specimen on the slide due to the irradiated light. The imaging unit is a camera provided in a microscope which includes a CCD (Charge Coupled Device) sensor and captures an image on an image forming face formed by the image forming unit to generate digital image data of the microscopic image. The communicator I/F transmits the image data of the generated microscopic image to the image processing device 2A. In this embodiment, the microscopic image acquiring device 1A includes a bright field unit which is a combination of the irradiating unit and the image forming unit suitable for bright field observation. The microscopic image acquiring device 1A may include a fluorescent unit which is a combination of the irradiating unit and the image forming unit suitable for fluorescence observation so that the bright field/fluorescence observation can be switched by switching the units.

The microscopic image acquiring device 1A is not limited to a microscope having a camera. For example, a virtual microscope slide creating apparatus which scans a slide on a slide fixing stage of a microscope and obtains a microscopic image of the entire tissue specimen may be used (for example, see Japanese Patent Application Laid-Open Publication No. 2002-514319). According to the virtual microscope slide creating apparatus, image data can be obtained with which the entire image of the tissue specimen on the slide can be viewed at once on a display.

The image processing device 2A analyzes the microscopic image transmitted from the microscopic image acquiring device 1A to identify a type of cell appearing in the tissue specimen of the observation target, and outputs the result.

FIG. 2 shows an example of a functional configuration of the image processing device 2A. As shown in FIG. 2, the image processing device 2A includes a controller 21, an operation interface 22, a display 23, a communicator I/F 24, a memory 25, and the like, and each unit is connected through a bus 26.

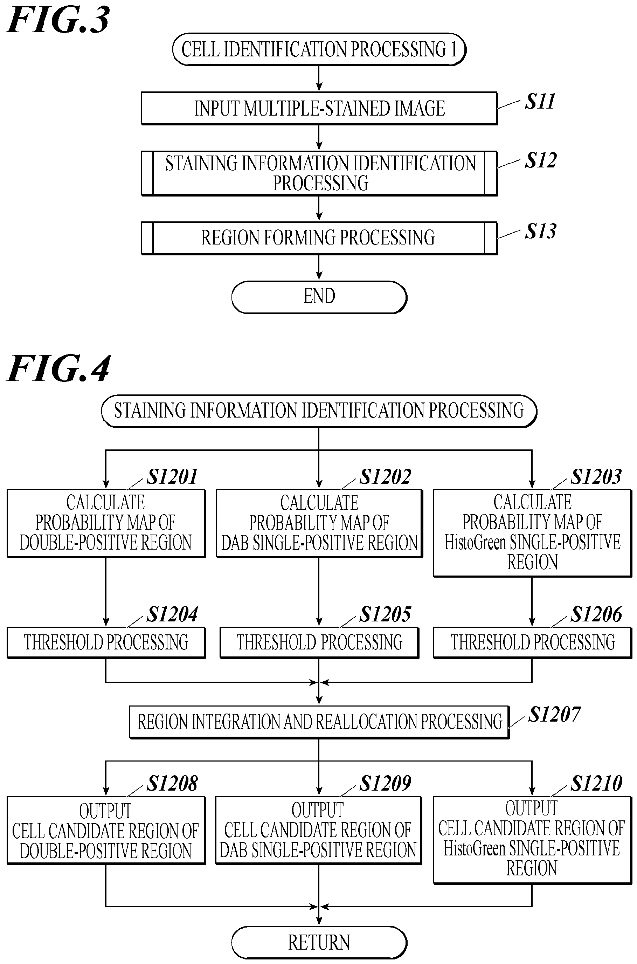

The controller 21 includes a CPU (Central Processing Unit), a RAM (Random Access Memory), and the like, performs various processing in coordination with various programs stored in the memory 25, and collectively controls the operation of the image processing device 2A. For example, the controller 21 executes cell identification processing 1 (see FIG. 3) and the like in coordination with programs stored in the memory 25, and functions as a staining information identifier and a region forming unit that execute staining information identification and region forming, respectively (and further as means for executing division information identification and division in the second embodiment).

The operation interface 22 includes a keyboard provided with character input keys, numeric input keys, and various function keys and a pointing device such as a mouse, and outputs depression signals of the pressed keys of the keyboard and operation signals of the mouse as the input signal to the controller 21.

The display 23 includes, for example, a monitor such as a CRT (Cathode Ray Tube), an LCD (Liquid Crystal Display), and the like, and displays various screens according to an instruction of a display signal input from the controller 21. In this embodiment, the display 23 functions as an output means that outputs a cell region as a result of cell identification processing 1.

The communicator I/F 24 is an interface for transmitting and receiving data to and from external devices such as the microscopic image acquiring device 1A. The communicator I/F 24 functions as a means for inputting a bright field image.

The memory 25 includes, for example, an HDD (Hard Disk Drive), a nonvolatile semiconductor memory, and the like. The memory 25 stores various programs and various pieces of data as described above.

Other than the above, the image processing device 2A may include a LAN adaptor, a router, and the like, and may be connected to external devices through a communication network such as a LAN.

### ACQUIRING MULTIPLE-STAINED IMAGE

The image processing device 2A in the present embodiment performs analysis using a stained image of a tissue specimen transmitted from the microscopic image acquiring device 1A, in particular, a multiple-stained image obtained by staining a plurality of types of biological materials with a plurality of stains that produce different colors.

The multiple-stained image in the present embodiment is an image obtained by imaging, with the microscopic image acquiring device 1A, a tissue specimen in which two or more types of proteins specifically expressed in macrophages, for example, have been visualized with a plurality of stains. Two types arbitrarily selected from among proteins and genes may be used, and a tissue specimen in which biological materials such as a chromosome and endoplasmic reticulum have been visualized with stains may be used.

A method of acquiring a multiple-stained image will be described in detail together with stains to be used for acquiring this multiple-stained image, a method of staining a tissue specimen with the stains, and the like.

### PROTEINS AND THE LIKE

Proteins are arbitrarily selected from among proteins specifically expressed in macrophages, for example, and examples thereof include PD-1, CD163, CD204, CD68, Iba1, CD11c, CD206, CD80, CD86, CD163, CD181, CD197, iNOS, Arginase1, CD38, Egr2, and the like.

At least one of two or more types of macrophage proteins targeted for staining is preferably a protein (for example, CD163, CD204, or the like) specifically expressed in M2 macrophages. In a case of using a tumor tissue as a tissue specimen, proteins specifically expressed in tumor-associated macrophages (TAM) are preferable.

A selection may be arbitrarily made from among biological materials expressed and secreted in cancer cells and the like, and examples thereof include PDL1, CTLA4, CD8, CD30, CD48, CD59, IDO, TDO, CSF-1R, HDAC, CXCR4, FLT-3, TIGIT, INF-α, INF-β, INF-ω, INF-ε, INF-κ, INF-γ, INF-λCSF, EPO, EGF, FGF, PDGF, HGF, TGF, CD3, CD4, CD25, CD28, CD80, CD86, CD160, CD57, OX40 (CD134), OX40L (CD252), ICOS (CD278), ICOSL (CD275), CD155, CD226, CD112, CD27, CD70, 4-1BB (CD137), 4-1BBL (CD137L), GITR (CD357), GITRL, BTLA (CD272), HVEM (CD270), TIM-3, Galectin-9, LAG-3 (CD223), B7-H3 (CD276), B7-H4, B7-H5, CD40, CD40L, PD-1, PD-L2, 2B4 (CD244), KLRG-1, E-Cadherin, N-Cadherin, R-Cadherin, CD68, CD163, and CSF1-R.

Genes may be DNAs, or may be RNAs such as mRNA, tRNA, miRNA (miR4717 or the like), siRNA, and non-cording-RNA.

In the present embodiment, CD68 as a protein expressed both in the M1 macrophages and M2 macrophages and CD163 as a protein specifically expressed in the M2 macrophages (or TAM) shall be stained using the following stains, respectively. Therefore, it is determined that a cell in which both CD68 and CD163 are expressed is the M2 macrophage (or TAM), and a cell in which only CD68 is expressed is the M1 macrophage.

### STAINS

The tissue specimen is preferably stained by bringing the tissue specimen into contact with a labeling substance to directly or indirectly bind the labeling substance with a macrophage protein targeted for staining, and, for example, immunostaining is preferable which is performed by causing a labeling antibody obtained by binding a labeling substance with an antibody that directly or indirectly binds with the macrophage protein to react with the tissue specimen.

The tissue specimen is preferably stained through dye staining. Dye staining is not particularly limited as long as a technique of staining each macrophage protein with a dye that enables bright field observation is used. For example, an enzyme antibody method of staining a target substance by binding a labeling substance (enzyme) with a macrophage protein targeted for staining by an arbitrary method and adding a dye (substrate) that produces a color through an enzyme substrate reaction to precipitate the dye in the tissue specimen can be used.

For example, immunostaining is preferable which is performed by adding, to a tissue specimen in which a labeling antibody obtained by binding an enzyme with an antibody that directly or indirectly binds with a target protein has been reacted in advance, a dye which is a substrate of that enzyme. The enzyme includes peroxidase and alkaline phosphatase, and the dye includes 3,3'-diaminobenzidine (DAB), HistoGreen, TMB, Betazoid DAB, CardassianDAB, Bajoran Purple, Vina Green, Romulin AEC, Ferangi Blue, VulcanFast Red, Warp Red, and the like. In a case of performing staining with DAB, staining is preferably performed before staining with another stain in view of dye stability.

In the present embodiment, CD68 and CD163 are stained with DAB and HistoGreen, respectively.

### TISSUE SPECIMEN

The tissue specimen refers to a tissue section collected from a subject, or cells obtained by culturing cells included in a tissue collected from a subject. In the present embodiment, a tissue section collected from a tumor tissue shall be used. The tissue specimen generally has the form of a specimen slide on which a tissue section or cells are placed, as commonly used in a case of assessing an expression level of a target protein through immunostaining, or the like.

The method of making the tissue specimen is not particularly limited. The tissue specimen is generally obtained by, for example, cutting a tissue sample into sections of 3 to 4 µm, the tissue sample being made by fixing a tissue section collected from a subject using formalin or the like, performing xylene processing after dewatering with alcohol, immersing the tissue section in high-temperature paraffin to perform paraffin embedding. The tissue section is placed on a slide glass and dried to make a specimen slide.

### STAINING METHOD

The staining method for the tissue specimen will be described. The staining method described below can be applied not only to the tissue section but also to cells.

### i) REMOVING PARAFFIN

A tissue specimen is immersed in a container with xylene, and paraffin is removed. The temperature is not particularly limited, and the processing can be performed at room temperature. Preferably, the immersing time is 3 minutes or more and 30 minutes or less. The xylene can be changed during the immersion as necessary.

Next, the tissue specimen is immersed in a container with ethanol, and the xylene is removed. The temperature is not particularly limited, and the processing can be performed at room temperature. Preferably, the immersing time is 3 minutes or more to 30 minutes or less. The ethanol can be changed during the immersion as necessary.

Next, the tissue specimen is immersed in a container with water to remove the ethanol. The temperature is not particularly limited, and the processing can be performed at room temperature. Preferably, the immersing time is 3 minutes or more and 30 minutes or less. The water can be changed during the immersion as necessary.

### ii) ACTIVATING PROCESSING

Activating processing of the target biological material in the tissue section is performed according to known methods. The activating conditions are not specifically set, and examples of liquid for activation that can be used include, 0.01 M citric acid buffered solution (pH 6.0), 1 mM EDTA solution (pH 8.0), 5% urea, 0.1 M tris-hydrochloric acid buffered solution. Examples of the heating device that can be used include autoclave, microwave, pressure pan, water bath, and the like. The temperature is not particularly limited, and the processing can be performed at room temperature. The processing can be performed at a temperature of 50 to 130 °C and the amount of time that the processing is performed can be 5 to 30 minutes.

Next, the tissue specimen after activating processing is immersed in the container with Phosphate Buffered Saline (PBS), and cleaning is performed. The temperature is not limited, and the processing can be performed at room temperature. Preferably, the immersing time is 3 minutes or more to 30 minutes or less. The PBS can be changed during the immersion as necessary.

### iii) BLOCKING PROCESSING

After the activating processing and before performing each staining, processing such as blocking is preferably performed according to necessity for the purpose of reducing background noise or the like. For example, dropping a surfactant such as BSA (Bovine serum albumin)-containing PBS or Tween20 as a blocking agent restrains an antibody from non-specifically adsorbing to the tissue specimen. For example, in a case of using an enzyme substrate reaction of peroxidase in dye staining, or the like, processing such as peroxidase blocking by means of hydrogen peroxide is preferably performed in order to prevent a non-specific color reaction due to endogenous peroxidase.

After performing these kinds of processing, cleaning with PBS or the like is preferably performed. Cleaning conditions can be adjusted as appropriate in accordance with the processing having been performed, and can be performed at room temperature for 3 minutes or more and 30 minutes or less, for example.

### iv) STAINING PROCESSING

The tissue specimen after cleaning is subjected to dye staining.

In immunostaining, a labeling antibody obtained by directly or indirectly binding an antibody capable of directly or indirectly binding with a macrophage protein targeted for detection and a labeling substance is dispersed in an appropriate medium, and the medium is placed on a tissue specimen such as a tissue section to cause reaction with a target biological material. The target macrophage protein is thus stained.

The labeling antibody may be obtained by binding a labeling substance with a primary antibody, or may be obtained by binding a labeling substance with a secondary antibody. In the case where the labeling antibody is obtained by binding a labeling substance with the primary antibody, the labeling antibody directly binds with macrophage proteins in the tissue specimen to stain them. In the case where the labeling antibody is obtained by binding a labeling substance with the secondary antibody, the labeling antibody binds with the primary antibody bound in advance with macrophage proteins in the tissue specimen to stain the macrophage proteins in the tissue specimen.

The antibody and the labeling substance may bind directly or indirectly. For example, one of staining modes in which the labeling antibody is the secondary antibody, and the secondary antibody and the labeling substance bind indirectly is expressed as: [tissue specimen (target protein)] ... [primary antibody (probe)] ... [secondary antibody]-[biotin]/[avidin]-[labeling substance].

An embodiment in which the notation "..." represents binding through an antigen-antibody reaction, the hyphen "-" represents binding through a covalent bond which may be mediated by linker molecules according to necessity, and the slash "/" represents binding through an avidin-biotin reaction is provided. Such staining is performed by, for example, immersing at first a tissue specimen in a solution of the primary antibody, then immersing the tissue specimen in a solution of a secondary antibody-biotin bound body, and finally immersing the tissue section in a solution of avidin-labeling substance.

As the primary antibody, an antibody (IgG) that specifically recognizes a protein which is a target biological material as an antigen and binds therewith is used.

As the secondary antibody, an antibody (IgG) that specifically recognizes the primary antibody as an antigen and binds therewith is used.

Both the primary antibody and the secondary antibody may be polyclonal antibodies; however, in view of stability of quantification, monoclonal antibodies are preferable. The type of an animal (immunized animal) which produces antibodies is not particularly limited, and a selection may be made from among mouse, rat, guinea pig, rabbit, goat, sheep, and the like similarly to traditional cases.

In conjunction with the above-described dye staining, fluorescent staining of subjecting an arbitrary substance to fluorescent labeling may be performed. For example, an arbitrary gene can be stained through fluorescence in situ hybridization (FISH) through use of a fluorescent dye.

### v) POSTPROCESSING

The tissue specimen having undergone the staining is preferably subjected to processing such as immobilization and dehydration, clearing, and sealing so as to be suitable for image capturing for signal measurement described above, or the like.

In the immobilization and dehydration processing, the tissue specimen may be immersed in a fixing processing liquid (a cross-linking agent such as formalin, paraformaldehyde, glutaraldehyde, acetone, ethanol, or methanol). In clearing, the specimen slide having undergone the immobilization and dehydration processing may be immersed in a clearing liquid (such as xylene). In the sealing processing, the specimen slide having undergone the clearing processing may be immersed in a sealing liquid. Conditions for performing these kinds of processing, the temperature and immersing time when immersing the specimen slide in a predetermined processing liquid, for example, are adjusted as appropriate according to traditional immunostaining such that an appropriate signal is obtained.

### OPERATIONS OF IMAGE PROCESSING SYSTEM 100 (INCLUDING IMAGE PROCESSING METHOD)

Operations of acquiring a multiple-stained image described above and performing analysis in the image processing system 100 will be described.

First, an operator prepares a tissue specimen in which CD68 and CD 163 have been stained with DAB and HistoGreen, respectively, through the above-described method.

Subsequently, the microscopic image acquiring device 1A acquires a multiple-stained image through the following steps (a1) to (a3).
(a1) The operator puts the tissue specimen on a slide, and places the slide on a slide fixing stage of the microscopic image acquiring device 1A.
(a2) The bright field unit is set, magnification and focus are adjusted, and an observation target region in the tissue is positioned in a visual field.
(a3) The imaging unit performs imaging to generate image data of the multiple-stained image, and the image data is transmitted to the image processing device 2A.

In the case of subjecting the tissue specimen to fluorescent staining in addition to dye staining, a fluorescence image is acquired through the steps (a4) and (a5) after the step (a3).
(a4) The unit is changed to the fluorescent unit.
(a5) The imaging unit performs imaging without changing the visual field and the magnification to generate image data of the fluorescence image, and the image data is transmitted to the image processing device 2A.

In the image processing device 2A, cell identification processing 1 is performed based on a bright field image.

FIG. 3 shows a flowchart of cell identification processing 1 executed in the image processing device 2A. The controller 21 executes cell identification processing 1 shown in FIG. 3 in coordination with programs stored in the memory 25.

First, the communicator I/F 24 inputs the multiple-stained image from the microscopic image acquiring device 1A (Step S11). The controller 21 executes staining information identification processing (Step S12: staining information identification).

FIG. 4 shows a detailed flow of staining information identification processing of Step S12. The controller 21 performs processing of Step S12 in coordination with programs stored in the memory 25.

In the staining information identification processing, first, a probability map of each of a double-positive region (Step S1201), a DAB single-positive region (Step S1202), and a HistoGreen single-positive region (Step S1203) is calculated under the control of the controller 21.

The double-positive region is a region considered as including double-positive cells, that is, TAM, in which color production of both of DAB and HistoGreen is confirmed in the multiple-stained image. DAB that labels CD68 colors the cytoplasm, and HistoGreen that labels CD163 colors the cell membrane. Their color producing positions do not completely agree with each other, and the stains fragmentarily stain sections different from each other. A region in which color production of both the stains is confirmed in proximity to each other is highly likely to include double-positive cells.

The DAB single-positive region and the HistoGreen single-positive region are regions in which color production of only one of DAB and HistoGreen is confirmed, respectively, and they are highly likely to include DAB single-positive cells and HistoGreen single-positive cells, respectively.

The multiple-stained image includes a mix of these regions. By correctly determining in which of the regions each stained region is included, the type of cells included in each of the stained regions is identified. The probability map is obtained by calculating a probability as to which type of cells each of the stained regions includes, and is shown in a mode that can be perceived spatially.

A method of producing a probability map will be described.

The probability map is produced as an output of a convolutional neural network (CNN) trained based on image patches including local regions having a predetermined size, for example.

Similarly to traditional deep learning, CNN performs supervised learning using a large amount of images in which an identification target appears as learning images to automatically learn features of the identification target appearing in the images in the neural network.

CNN is trained as follows, for example. The neural network in CNN generally has a hierarchical structure, and has an input layer, a convolution layer, a pooling layer, a fully connected layer, an output layer, and the like. The convolution layer and the pooling layer are repeated alternately several times for input learning images to extract features of the identification target appearing in the input images, and finally, the identification target appearing in the images is identified in the fully connected layer. Errors between an identification result obtained in this manner and labels (ground truth) attached to the learning images are obtained, and the neural network is reversely propagated to change parameters in each layer of CNN (backpropagation). By changing parameters in each layer in this manner to change data to be transferred, the accuracy of identification is improved so that various identification targets can be identified.

In the present embodiment, CNN is trained by using images in which a double-positive cell, DAB single-positive cell, and HistoGreen single-positive cell have been identified in advance as learning target images by an intellectual such as a pathologist, for example, and which are provided with class labels corresponding to the respective cell types. Regions including peripheral pixels around pixels corresponding to these labels, that is, local regions including any of the double-positive cell, DAB single-positive cell, and HistoGreen single-positive cell are extracted from the learning target images as image patches. CNN is trained by subjecting these image patches to operations in the respective layers as described above, identifying which of the double-positive cell, DAB single-positive cell, and HistoGreen single-positive cell each of the image patches is, obtaining errors between the identification result and labels (ground truth) provided in advance, and adjusting parameters through backpropagation.

CNN trained as described above identifies the cell types in a multiple-stained image having been input taking a spatial correlation between stains in a local region and relative positional relationship between cell components into consideration.

The spatial correlation between stains refers to a positional relationship between stains, such as the presence of, close to a stain of a certain color, a stain of another color. For example, since double-positive cells are stained with two types of stains, if color production of both the stains is confirmed in a local region including a cell of interest, the cell is highly likely to be a double-positive cell. Alternatively, if color production of only a single stain is confirmed in a local region including a cell of interest, the cell is highly likely to be a single-positive cell. The positional relationship between cell components refers to an obvious containment relationship between cell components, such as the presence of the cytoplasm around the nucleus, and further outside thereof the presence of the cell membrane.

According to the example of the present embodiment, if the green color of HistoGreen that colors the cell membrane is confirmed in a manner surrounding the brownish color of DAB that colors the cytoplasm in a local region (double-positive region) in which both stains of DAB and HistoGreen are located in proximity to each other, the stained region surrounded by the green color of HistoGreen is determined as being a single double-positive cell.

That is, merely by extracting a region stained with any stain simply as a cell region as in the traditional technology, it is difficult to identify cell types based on the difference in stained state. In the present embodiment, by considering the spatial correlation between stains and the relative positional relationship between cell components as described above, complicated identification targets like fragmentarily stained cells, such as double-positive cells and single-positive cells, are extracted from a multiple-stained image stained with a plurality of stains, and the types of these cells are identified based on the difference in stained state.

Specifically, CNN repeats filter processing in the convolution layer and image zoom-out processing in the pooling layer alternately several times for the multiple-stained image input in Step S11 to extract features from the multiple-stained image. Each layer outputs feature maps in which extracted features are arrayed two-dimensionally by the number of filters. The output feature maps output a fully connected map in the fully connected layer, and a probabilistic label is generated per cell region. That is, the probability map is obtained by calculating an existence probability of each cell type (double-positive cell, DAB single-positive cell, and HistoGreen single-positive cell) for each position in the multiple-stained image, and spatially connecting and arraying them. Each position described above may be a range having an extension and including a plurality of pixels in the multiple-stained image, or may be a single pixel. Therefore, the probability map is supposed to be an output result obtained by calculating the existence probability for every two pixels in the multiple-stained image, an output result obtained by calculating the existence probability for each pixel, or the like.

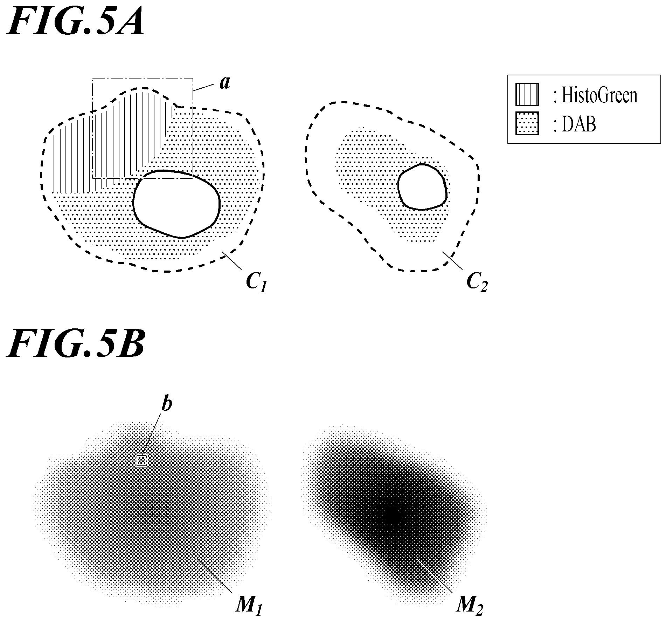

FIGs. 5A and 5B show conceptual diagrams of probability maps. For example, in a case where a multiple-stained image in which a double-positive cell C₁ and a DAB single-positive cell C₂ as shown in FIG. 5A are located is input to the image processing device 2A, a filter indicated by a is placed on the whole image to extract features by CNN.

Based on the extracted features, probability maps of the respective cell types, such as a probability map M₁ of the double-positive cell and a probability map M₂ of the DAB single-positive cell shown in FIG. 5B, are output as monochrome masks. In the drawing, b corresponds to the existence probability in the region on which the filter a has been placed. By spatially connecting these existence probabilities over the whole multiple-stained image, the probability map is generated.

In the probability map, a calculated existence probability is expressed by a color. The method of expression is not limited. For example, the probability map may be a map representing the inside and outside of a cell by a binary value of 1/0, or may be a map in accordance with a concept other than a directly existing object, such as a map obtained by converting the distance from the cell center into color intensity or a map obtained by converting the distance from the cell boundary into color intensity. These are merely examples, and the above-described examples are not limitations, but an arbitrary design can be made.

The controller 21 subjects each of the probability maps calculated in the above manner to threshold processing (Step S1204, Step S1205, and Step S1206). The threshold processing is processing of dropping a value less than a threshold previously set by the operator in the probability map. Accordingly, a probability map in which only a region having an existence probability more than or equal to a certain value is displayed for each region is output.

The controller 21 then executes region integration and reallocation processing (Step S1207). The region integration and reallocation processing is processing of determining which cell region each stained region in the multiple-stained image is, based on the probability maps.

In the region integration and reallocation processing, the double-positive region, DAB single-positive region, and HistoGreen single-positive region calculated respectively in Step S1201 to Step S1206 are integrated first. Merely by subjecting the probability maps of the respective regions to threshold processing in Step S1204 to Step S1206, the probability maps will partially overlap with each other. In order to isolate such a common portion, region forming is performed such that each cell candidate region is output exclusively. Ranking cell candidate regions is effective for this, and a method such as displaying a double-positive cell with priority over a single-positive cell, for example, can be used.

When the integration and reallocation processing is completed in the above manner, a cell candidate region of the double-positive region (Step S1208), a cell candidate region of the DAB single-positive region (Step S1209), and a cell candidate region of the HistoGreen single-positive region (step S1210) are output respectively, and the staining information identification processing is completed.

Returning to the flowchart of FIG. 3, the controller 21 executes region forming processing (Step S13: region forming).

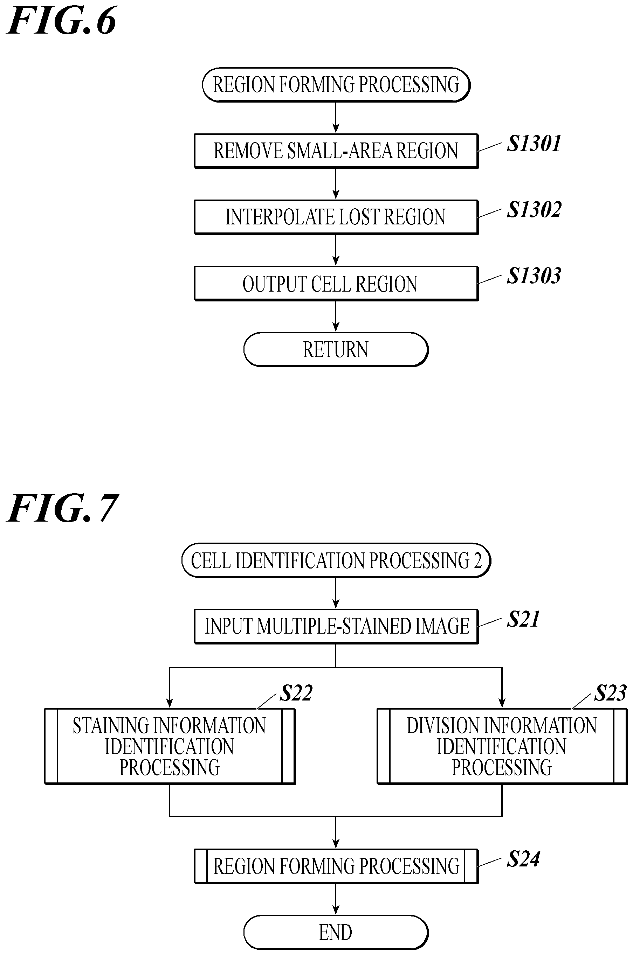

FIG. 6 shows a detailed flow of processing of Step S13. The controller 21 performs processing of Step S13 in coordination with programs stored in the memory 25.

In the region forming processing, the controller 21 first removes a small-area region whose area is less than a certain value in each cell candidate region (Step S1301). Removal of the small-area region is performed by opening processing on a binary image, for example. The opening processing is processing of performing erosion processing and then dilation processing by the same number of times. The erosion processing is processing of replacing a target pixel with a black pixel when any of the pixels within the range of n x n pixels from the target pixel is black. The dilation processing is processing of replacing the target pixel with a white pixel when any of the pixels within the range of n x n pixels (n is an integer of 2 or more) from the target pixel is white. A cell candidate region from which minute regions such as noise have been removed by the opening processing is output.

Subsequently, the controller 21 interpolates a lost region (hole region) in each cell candidate region (Step S1302). Interpolation of a lost region is performed by performing closing processing on the binary image, for example. The closing processing is processing of performing dilation processing and then erosion processing by the same number of times. A cell candidate region in which a lost region has been interpolated by the closing processing is output.

Noise, losses, and the like in the cell regions are removed by the above processing, and an identification result of final cell regions is output (Step S1303). The region forming processing is completed.

When the region forming processing is completed, cell identification processing 1 of FIG. 3 is completed.

As described above, in the image processing method according to the present embodiment, cell types are identified in a multiple-stained image obtained by imaging a tissue specimen subjected to multiple staining with a plurality of stains that produce different colors, based on the difference in stained state between cells. In staining information identification processing, the stained state of cells is identified based on a spatial correlation between the stains and a relative positional relationship between cell components, and a probability map representing the existence probability is output for each cell type. That is, in the image processing method according to the present embodiment, complicated identification targets such as fragmentarily stained cells are extracted from a multiple-stained image stained with a plurality of stains.

In the image processing method according to the present embodiment, a tissue specimen including cells in which a plurality of different biological materials have been stained with stains that produce different colors, respectively, is used. This enables cell types to be identified based on the difference in stained state.

In the image processing method according to the present embodiment, cell candidate regions roughly extracted in the staining information identification processing are subjected to the region forming processing of removing a small-area region and/or interpolating a lost region. This enables cell regions to be output in a more complete form.

In the above-described embodiment, removal of a small-area region and interpolation of a lost region are both performed as the region forming processing. However, these kinds of processing should only be performed according to necessity, and only either one of them may be executed.

### SECOND EMBODIMENT

The second embodiment for carrying out the present invention will be described with reference to the drawings. Components identical to those of the first embodiment will be given identical reference numerals, and description thereof will be omitted.

In the second embodiment, the cell identification processing includes division of dividing a cell candidate region obtained by the staining information identification processing into individual cells.

In the staining information identification processing, a candidate region of each of a double-positive cell, DAB single-positive cell, and HistoGreen single-positive cell is output. If a plurality of cells are located in proximity to each other in each region, these cells may be connected and output as one region. Therefore, in the cell identification processing in the present embodiment, division processing for dividing such a cell candidate region formed with a plurality of cells connected to each other into individual cells and outputting them is executed, and division information identification processing for specifying a division reference to be used as a reference in the division processing is included.

### OPERATIONS OF IMAGE PROCESSING SYSTEM 100 (INCLUDING IMAGE PROCESSING METHOD)

Operations of acquiring the multiple-stained image described above and performing analysis in the image processing system 100 will be described. The method of acquiring data of a multiple-stained image performed by an operator is similar to that of the first embodiment, and description thereof will be omitted.

FIG. 7 shows a flowchart of cell identification processing 2 executed in the image processing device 2A. The controller 21 executes cell identification processing 2 shown in FIG. 7 in coordination with programs stored in the memory 25.

First, the communicator I/F 24 inputs the multiple-stained image from the microscopic image acquiring device 1A (Step S21). The controller 21 executes staining information identification processing (Step S22: staining information identification). The processing of Step S22 is identical to the staining information identification processing in the first embodiment, and description thereof will be omitted. That is, the cell candidate region of each of the double-positive region, DAB single-positive region, and HistoGreen single-positive region is output through the processing of Step S22.

Upon receipt of the multiple-stained image, the controller 21 executes division information identification processing (Step S23: division information identification).

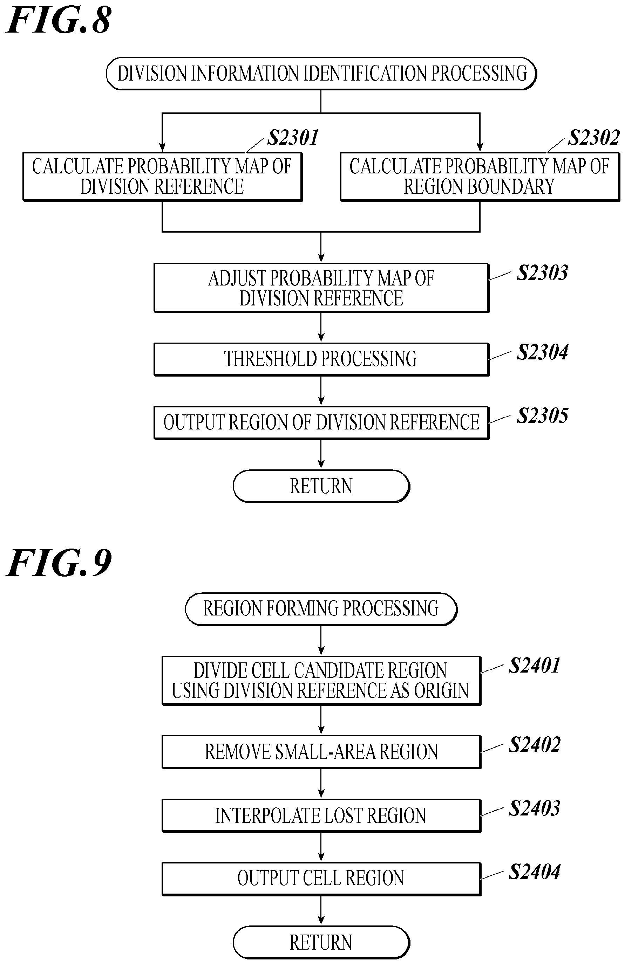

FIG. 8 shows a detailed flow of processing of Step S23. The controller 21 executes the processing of Step S23 in coordination with programs stored in the memory 25.

In the division information identification processing, the probability map of a division reference is calculated first under the control of the controller 21 (Step S2301).

The division reference refers to an object or a region used as a reference when dividing a single continuous region into a plurality of regions. Specifically, cell components such as the nucleus, cytoplasm, and cell membrane are suitable as division references, and in a case of using them as division references, they can be stained with individual stains.

However, the division references are not limited to such certain cell components. The division references are preferably set considering the positional relationship between cell components, such as separation of cells with a biological structure, that is, the cell membrane, presence of the cytoplasm and nucleus on the inner side of the cell membrane, or inclusion of the nucleus by the cytoplasm, and the spatial correlation between stains that stain them. An unstained region surrounded by a region stained with stains, that is, a region in which the nucleus or the like is considered as being present, may be set as a division reference.

Another stain (in the present embodiment, DAB) contained in one stain (in the present embodiment, HistoGreen) can be used as a division reference. In Step S2301, the DAB stained region is set as a division reference, and the existence probability thereof is calculated as a probability map.

The probability map of the division reference can be calculated by CNN similarly to the probability map of the cell candidate region. That is, in the case of using the DAB stained region as a division reference, it is effective to calculate the probability map of the division reference using CNN trained so as to be capable of identifying, in the multiple-stained image, the DAB stained region surrounded by the above-described HistoGreen stained region.

Upon receipt of input of the multiple-stained image, the controller 21 calculates the probability map of a region boundary (Step S2302). The region boundary refers to a boundary portion between one division reference and a region outside thereof (a peripheral stained region, an adjacent division reference, or the like). Merely by performing threshold processing, the probability map of the division reference calculated in Step S2301 may be output with division references being connected to each other. Thus, by adjusting the probability map of the division reference with the probability map of the region boundary, the individual division references are separated.

The probability map of the region boundary is calculated by, for example, CNN trained so as to be capable of identifying the region boundary of the division reference in the multiple-stained image.

When the processing of Step S2301 and the processing of Step S2302 are completed, the controller 21 adjusts the probability map of the division reference (Step S2303). Specifically, the probability map is adjusted by subtracting, from a probability (numeric value) in each pixel in the probability map of the division reference, a probability in a pixel in a corresponding probability map of the region boundary multiplied by an arbitrarily set weight.

Then, the adjusted probability map of the division reference is subjected to the threshold processing (Step S2304). A final region of the division reference is output (Step S2305). The division information identification processing is completed.

Returning to the flowchart of FIG. 7, the controller 21 executes region forming processing (Step S24: region forming).

FIG. 9 shows a detailed flow of processing of Step S24. The controller 21 performs processing of Step S24 in coordination with programs stored in the memory 25.

In the region forming processing, under the control of the controller 21, each cell candidate region output in the staining information identification processing is first subjected to division processing using the division reference specified in the division information identification processing as an origin (Step S2401: division). A marker-controlled watershed algorithm, for example, can be applied to this division processing.

The watershed algorithm is a technique of, when assuming the brightness gradient of an image as a topographical map of mountains and valleys and imagining flowing water thereto, determining a watershed for storing water as the boundary of regions.

In the present embodiment, in a case of dividing each cell candidate region by a marker-controlled watershed algorithm using the DAB stained region (division reference) specified in the division information identification processing as a marker, the edge portion of a cell candidate region including a plurality of DAB stained regions is assumed as a mountain, and low-bright pixels are removed in a manner flooding the cell candidate region while keeping the water level equal in the whole image to reduce the cell candidate region until a state in which each candidate region of the nucleus is included in a fragment of a single cell candidate region is brought about. Then, the fragment of the cell candidate region is enlarged to the cell candidate region prior to the start of reduction, and pixels in which enlarged cell candidate regions abut on each other are used as the boundary. Individual cells in the cell candidate region are thus separated.

When the cell candidate region is divided into individual cells, the controller 21 executes removal of a small-area region (Step S2402) and interpolation of a lost region (Step S2403). These kinds of processing are similar to those of the first embodiment, and description thereof will be omitted.

After the above processing, individual cells of a final cell region, that is, the cell candidate region are separated and output (Step S2404). The region forming processing is completed.

When the region forming processing is completed, cell identification processing 2 of FIG. 7 is completed.

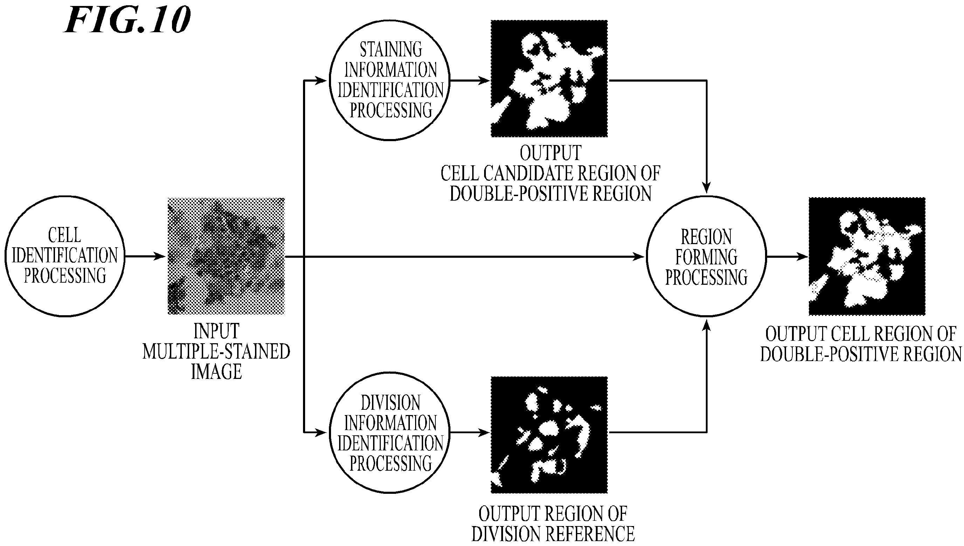

FIG. 10 shows an example of a cell region of a double-positive region extracted using the image processing system 100 according to the present embodiment.

As shown in FIG. 10, a cell candidate region of the double-positive region is roughly extracted from the multiple-stained image through the staining information identification processing. A DAB stained region is extracted as a division reference through the division information identification processing. Based on them, a final cell region is output in a manner divided into individual cells output continuously through the region forming processing.

As described above, the image processing method according to the present embodiment executes the division information identification processing for specifying the division reference. Accordingly, division processing for dividing a continuously output cell candidate region into cells is executed. Furthermore, in the division information identification processing, the probability map of the division reference is generated, and this is adjusted by the probability map of the region boundary, so that the division reference is specified more correctly.

As described above, the division reference may be the nucleus located in a region stained with a stain, or may be a cell component other than the nucleus. Alternatively, an unstained region surrounded by a stained region may be used as a division reference. That is, the division processing can be applied to every type of cells such as a cell without nucleus and a cell having a plurality of nuclei.

The above-described embodiment uses the marker-controlled watershed algorithm for division processing of the cell candidate region. However, this is not a limitation. Graphcut, for example, may be used instead.

### OTHER EMBODIMENTS

The description in the above embodiments provides preferable examples of the present invention, but the present invention is not limited thereto.

The above-described embodiments have been described using the case of staining CD68 and CD163 with DAB and HistoGreen, respectively, as an example, but this is not a limitation. For example, the present invention is also applicable to regulatory T cells in which C25 and FoxP3 have been stained, cancer-associated fibroblasts (CAFs) in which FAP, αSMA, and vimentin have been stained, B cells in which CD19 and CD20 have been stained, and the like.

The above description discloses an example which uses an HDD, a semiconductor nonvolatile memory, or the like as the computer readable medium of the program of the present invention. However, the present invention is not limited to the above. A portable recording medium such as a CD-ROM, and the like can be applied as other computer readable media. A carrier wave can be applied as the medium which provides the data of the program of the present invention through a communication line.

The image processing device does not necessarily consist of a single device, but may consist of a combination of specialized devices for respective components, such as a staining information identifier, a region forming unit, and a division information identifier that execute the staining information identification, region forming, and division information identification, respectively.

Other than the above, the detailed configuration and the detailed operation of each device composing the image processing system 100 can be suitably changed within the scope of the present invention.

### INDUSTRIAL APPLICABILITY

The present invention may be applied to an image processing method, an image processing device, and a program.

### REFERENCE SIGNS LIST

- 1A: Microscopic image acquiring device
- 2A: Image processing device
- 21: Controller (staining information identifier, region forming unit)
- 22: Operation interface
- 23: Display
- 24: Communicator I/F
- 25: Memory
- 26: Bus
- 3A: Cable
- 100: Image processing system

## Claims

1. An image processing method for identifying cell types in a multiple-stained image obtained by imaging a tissue specimen subjected to multiple staining with a plurality of stains that produce different colors, based on a difference in stained state between cells, comprising:
identifying staining information by identifying a cell candidate region in which cells in a specific stained state exist in the multiple-stained image; and
forming a region by subjecting the cell candidate region to predetermined region forming processing to form a cell region in which the cells in the specific stained state have been identified,
wherein identifying the staining information includes:
identifying a state of the cells in the multiple-stained image based on:
a spatial correlation between the stains representing whether staining of a pixel, positioned in proximity to a pixel detected as having been stained with one stain, with another stain has been detected; and
a relative positional relationship between cell components; and
calculating existence probabilities of the cells in the specific stained state for respective positions in the multiple-stained image, and outputting a probability map obtained by spatially connecting the existence probabilities.

2. The image processing method according to claim 1, wherein the tissue specimen includes a cell in which a plurality of different biological materials have been stained with stains that produce different colors, respectively.

3. The image processing method according to claim 1 or 2, wherein forming the region includes, as the predetermined region forming processing, subjecting the cell candidate region to removal of a small-area region and/or interpolation of a lost region.

4. The image processing method according to any one of claims 1 to 3, comprising:
identifying division information by identifying a division reference for dividing the cell candidate region into predetermined regions,
wherein identifying the division information includes:
calculating existence probabilities of the division reference for respective positions in the multiple-stained image based on the spatial correlation between the plurality of stains and the relative positional relationship between the cell components; and
generating a probability map of the division reference obtained by spatially connecting the existence probabilities.

5. The image processing method according to claim 4, wherein the division reference is a nucleus located inside a region stained with the stains.

6. The image processing method according to claim 4, wherein the division reference is an unstained region located inside the cell candidate region and surrounded by a region stained with the stains.

7. The image processing method according to any one of claims 4 to 6, wherein forming the region includes, as the predetermined region forming processing, dividing the cell candidate region output in identifying the staining information using the division reference output in identifying the division information as a reference.

8. An image processing device that identifies cell types in a multiple-stained image obtained by imaging a tissue specimen subjected to multiple staining with a plurality of stains that produce different colors, based on a difference in stained state between cells, comprising:
a staining information identifier that identifies a cell candidate region in which cells in a specific stained state exist in the multiple-stained image; and
a region forming unit that subjects the cell candidate region to predetermined region forming processing to form a cell region in which the cells in the specific stained state have been identified,
wherein the staining information identifier
identifies the stained state of the cells in the multiple-stained image based on:
a spatial correlation between the stains representing whether staining of a pixel, positioned in proximity to a pixel detected as having been stained with one stain, with another stain has been detected; and
a relative positional relationship between cell components, and
calculates existence probabilities of the cells in the specific stained state for respective positions in the multiple-stained image, and outputs a probability map obtained by spatially connecting the existence probabilities.

9. A program that makes a computer in an image processing device that identifies cell types in a multiple-stained image obtained by imaging a tissue specimen subjected to multiple staining with a plurality of stains that produce different colors based on a difference in stained state between cells function as:
a staining information identifier that identifies a cell candidate region in which cells in a specific stained state exist in the multiple-stained image; and
a region forming unit that subjects the cell candidate region to predetermined region forming processing to form a cell region in which the cells in the specific stained state have been identified,
wherein the program causes the staining information identifier to
identify the stained state of the cells in the multiple-stained image based on:
a spatial correlation between the stains representing whether staining of a pixel, positioned in proximity to a pixel detected as having been stained with one stain, with another stain has been detected; and
a relative positional relationship between cell components, and
calculate existence probabilities of the cells in the specific stained state for respective positions in the multiple-stained image, and output a probability map obtained by spatially connecting the existence probabilities.
